# EUROPEAN PATENT SPECIFICATION

(11) **EP 3 087 970 B1**
(45) Date of publication and mention of the grant of the patent: **23.05.2018**
(21) Application number: 14874017.8
(22) Date of filing: 02.12.2014
(51) Int. Cl.: A61Q 17/04, A61Q 1/02, A61K 8/02, A61K 8/06, A61K 8/19, A61K 8/27, A61K 8/29, A61K 8/89, A61K 8/34

(54) **WATER-IN-OIL EMULSIFIED SUNSCREEN COSMETIC**
EMULGIERTES WASSER-IN-ÖL-KOSMETIKUM ALS SONNENSCHUTZ
PRODUIT COSMÉTIQUE D'ÉCRAN SOLAIRE ÉMULSIFIÉ D'HUILE-DANS-L'EAU

(30) Priority: 26.12.2013 JP 2013269122
(43) Date of publication of application: 02.11.2016
(73) Proprietor: Shiseido Company Ltd., Chuo-ku Tokyo 104-0061 (JP)
(72) Inventor: YAMAGUCHI Kazuhiro, Yokohama-shi Kanagawa 224-8558 (JP); TASHIRO Mayuri, Yokohama-shi Kanagawa 224-8558 (JP); SASAKI Kazutaka, Yokohama-shi Kanagawa 224-8558 (JP)
(74) Representative: Ter Meer Steinmeister & Partner
(86) International application number: PCT/JP2014/081812
(87) International publication number: WO 2015/098433

(56) References cited:
- EP-A1- 1 167 462
- EP-A2- 2 213 336
- WO-A1-00/42112
- WO-A1-97/45097
- WO-A1-2010/098099
- JP-A- S5 862 106
- JP-A- 2002 154 915
- JP-A- 2004 277 388
- JP-A- 2007 045 760
- JP-A- 2007 302 647
- US-A1- 2009 017 081
- US-A1- 2010 172 850
- US-A1- 2010 291 011
- US-A1- 2011 195 036
- US-A1- 2011 250 248
- KAZUYUKI BABA ET AL.: 'Saikin no Shigaisen Bogyozai no Kaihatsu' FRAGRANCE JOURNAL vol. 20, no. 6, June 1992, pages 35 - 40, XP008183772

## Description

### TECHNICAL FIELD

The present invention relates to a water-in-oil emulsified sunscreen cosmetic. More specifically, it relates to a water-in-oil emulsified sunscreen cosmetic that is applied on the face including the areas surrounding the eyes, wherein irritation to the eyes is minor, the UVB and UVA protection effect is high, and a superior makeup effect is also manifested.

### BACKGROUND ART

Because of their superior water resistance, water-in-oil emulsified compositions are used for many cosmetics including sunscreens and makeup products. Also, as the harm of the ultraviolet A (UVA) on the skin is understood more recently, sunscreen agents that not only block the UVB but also block the UVA are sought after.

In order to impart the sunscreen effect to sunscreen cosmetics, an ultraviolet scattering agent and/or an ultraviolet absorbent is blended in; for the purpose of better spreadability, liquid ultraviolet absorbents such as octylmethoxy cinnamate and/or octocrylene are widely used. Also, in order to block not only the UVB but also the UVA, zinc oxide and/or avobenzone are often used in a sunscreen cosmetic (for example, refer to Patent Documents 1-3 and Non-Patent Document 1).

Particularly with water-in-oil emulsified compositions with high water resistance, almost all the UVA ultraviolet absorbents are solid at room temperature, making them difficult to be dissolved for blending in, and therefore a frequently used technical method is to blend in liquid UVB absorbents such as octylmethoxy cinnamate or octocrylene, and zinc oxide powder as the UVA protection ingredient, or to blend in avobenzone, which has a high UVA absorbance per unit concentration, and liquid UVB absorbents such as octylmethoxy cinnamate or octocrylene.

### PRIOR ART DOCUMENTS

### PATENT DOCUMENTS

Patent Document 1: JP H5-504572 A
Patent Document 2: JP H4-226910 A
Patent Document 3: JP 4663879 B

### NON PATENT DOCUMENTS

Non-Patent Document 1: "Shin-Keshohin-Gaku [New Cosmetic Scienece] (second edition)" , Nanzando, 2001, pp. 478, Editor: Takeo Mitsui

### DISCLOSURE OF INVENTION

### TECHNICAL PROBLEM

However, the inventors of the present application, after years of sunscreen cosmetic research, discovered that sunscreen cosmetics containing representative UVB absorbents such as octylmethoxy cinnamate and octocrylene and/or the UVA absorbent avobenzone were disclosed many times in addition to the aforementioned prior art technical references but that said sunscreen cosmetics had a problem in that they caused irritation if they got in the eye.

Therefore, a sunscreen cosmetic containing octylmethoxy cinnamate, octocrylene, and/or avobenzone is not suitable for a sunscreen to be applied on the face, but, from the point of view of protecting the face from sunburn, the development of sunscreen cosmetics that sufficiently protect the face skin from the UVA and UVB ultraviolet is desired.

Based on the aforementioned point of view, the inventors of the present application conducted earnest research for the development of a sunscreen cosmetic to be applied on the face that irritates the eyes less, and as a result discovered that, by blending in a combination of titanium dioxide and zinc oxide having a specific particle size, a sufficiently high UVA and UVB ultraviolet protection effect could be obtained and that a sunscreen cosmetic that did not irritate the eyes and manifested a superior makeup effect with a natural skin color finish could be obtained, thus completing the present invention.

The object of the present invention is to provide a sunscreen cosmetic that manifests a sufficiently high UVA and UVB ultraviolet protection effect without blending in octylmethoxy cinnamate, octocrylene, and/or avobenzone, does not irritate the eyes, and manifests a superior makeup effect.

### TECHNICAL SOLUTION

That is, the present invention provides a water-in-oil emulsified sunscreen cosmetic comprising the following ingredients (a), (b), (c), and (d) in the outer phase, as well as (e) and (f), but not comprising octylmethoxy cinnamate, octocrylene, or avobenzone,
(a) 5-15 wt% of hydrophobized rutile type crystallized titanium dioxide having an average particle size of 30-80 nm
(b) 0.1-10 wt% of iron oxide
(c) 5-15 wt% of hydrophobized zinc oxide having an average particle size of 20-80 nm
(d) 0-1.0 wt% of titanium dioxide for white pigment having an average particle size of 180 nm or more,
(e) 0.5-4 wt% of a surfactant,
(f) 5-30 wt% of water,
wherein (e) the surfactant is a polyoxyethylene methylpolysiloxane copolymer, crystals of ingredient (a) are not cone-shaped but near spherical clumps, and the total blend ratio of ingredients (a) and (c) is 16-25 wt%.

### ADVANTAGEOUS EFFECTS OF THE INVENTION

The prominent effects of the sunscreen cosmetic of the present invention are as follows.
(1) It manifests a sufficiently high UVA and UVB ultraviolet protection effect without blending in octylmethoxy cinnamate, octocrylene, and/or avobenzone.
(2) Low irritation to the eyes. Therefore the sunscreen cosmetic of the present invention can be preferably used on the face including areas around the eyes.
(3) It has a superior makeup effect.

### BRIEF DESCRIPTION OF DRAWINGS

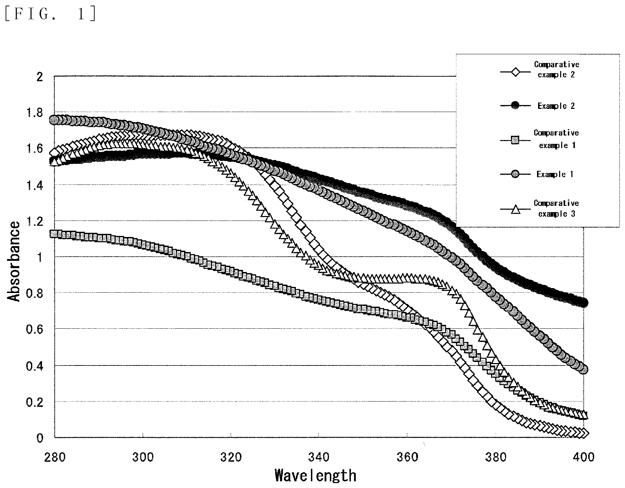

{FIG. 1} FIG. 1 is a graph that shows the UVA and UVB ultraviolet absorption effect of Examples and Comparative examples.

### MODES FOR CARRYING OUT THE INVENTION

The present invention is described in detail below.

### "(a) hydrophobized rutile type crystallized titanium dioxide having an average particle size of 30-80 nm"

The present invention uses (a) hydrophobized rutile type crystallized titanium dioxide having an average particle size of 30-80 nm.

In the present invention, the average particle size is measured with a conventional method such as a number average particle size (an average of random 50 particles) derived from image analysis of transmission electron microscope images.

The crystals of rutile type crystallized titanium dioxide used in the present invention are not cone-shaped but near spherical clumps.

The surface of the rutile type crystallized titanium dioxide used in the present invention is hydrophobized. Selection of the hydrophobizing treatment method is not limited in particular; a prior art method is used for the treatment. Examples include a treatment in which silicones such as methylhydrogen polysiloxane, methylhydrogen polysiloxane/dimethyl polysiloxane copolymer, and dimethyl polysiloxane are used, a treatment in which silane compounds such as octyltriethoxysilane and hexyltrimethoxysilane are used, a treatment in which a fatty acid such as palmitic acid or stearic acid is used, a metal soap treatment in which an alkali metal salt or alkaline earth metal salt of said fatty acid is used, and a fluorine treatment in which diethanolamine perfluoroalkylphosphate, perfluoroalkyltrimethoxysilane are used.

In the present invention it is particularly preferable to use aluminum stearate or octyltriethoxysilane for the hydrophobizing treatment.

In the present invention, the hydrophobized rutile type crystallized titanium dioxide is contained in the outer phase of the water-in-oil emulsified sunscreen cosmetic.

The blend ratio of the hydrophobized rutile type crystallized titanium dioxide having an average particle size of 30-80 nm is 5-15 wt%, preferably 6-12 wt%, and more preferably 7-10 wt%, relative to the total amount of the water-in-oil emulsified sunscreen cosmetic. If the blend ratio is less than 5 wt%, then a sufficient ultraviolet protection effect cannot be obtained, and if it is over 15 wt%, then the sunscreen cosmetic may become mealy and texture may become poor.

### "(b) Iron oxide"

In the present invention, iron oxide powder is used for ingredient (b). The type of the iron oxide (red iron oxide, yellow iron oxide, black iron oxide) and the average particle size are not limited in particular. In the present invention, the iron oxide is contained in the outer phase of the water-in-oil emulsified sunscreen cosmetic.

The blend ratio of the iron oxide is 0.1-10 wt%, preferably 0.5-5 wt%, and more preferably 1.1-3.3 wt%, relative to the total amount of the sunscreen cosmetic.

### "(c) Hydrophobized zinc oxide having an average particle size of 20-80 nm"

The present invention uses hydrophobized zinc oxide having an average particle size of 20-80 nm for ingredient (c).

The average particle size is measured with a conventional method such as a number average particle size (an average of random 50 particles) derived from image analysis of transmission electron microscope images.

In the present invention, the hydrophobized zinc oxide is contained in the outer phase of the water-in-oil emulsified sunscreen cosmetic.

The surface of the zinc oxide used in the present invention is hydrophobized. Selection of the hydrophobizing treatment method is not limited in particular; a prior art method is used for the treatment. Examples include a treatment in which silicones such as methylhydrogen polysiloxane, methylhydrogen polysiloxane/dimethyl polysiloxane copolymer, and dimethyl polysiloxane are used, a treatment in which silane compounds such as octyltriethoxysilane and hexyltrimethoxysilane are used, a treatment in which a fatty acid such as palmitic acid or stearic acid is used, a metal soap treatment in which an alkali metal salt or alkaline earth metal salt of said fatty acid is used, and a fluorine treatment in which diethanolamine perfluoroalkylphosphate, perfluoroalkyltrimethoxysilane are used. In the present invention, a hydrophobizing treatment using silicones or octyltriethoxysilane is preferable.

In the present invention, the hydrophobized zinc oxide is contained in the outer phase, i.e., the oil phase, of the water-in-oil emulsified sunscreen cosmetic.

The blend ratio of the hydrophobized zinc oxide having an average particle size of 20-80 nm is 5-15 wt%, preferably 7-13 wt%, relative to the total amount of the water-in-oil emulsified sunscreen cosmetic. If the blend ratio is less than 5 wt%, then a sufficient ultraviolet protection effect cannot be obtained, and if it is over 15 wt%, then the sunscreen cosmetic may become mealy and the texture may become poor.

Also, the total blend ratio of this and said hydrophobized rutile type crystallized titanium dioxide also influences the ultraviolet protection effect and texture.

The total blend ratio of ingredient (a) hydrophobized rutile type crystallized titanium dioxide and ingredient (c) hydrophobized zinc oxide is 16-25 wt%, relative to the total amount of the water-in-oil emulsified sunscreen cosmetic.

### "(d) Titanium dioxide for white pigment having an average particle size of 180 nm or more"

The present invention uses titanium dioxide for white pigment having an average particle size of 180 nm or more for ingredient (d).

The average particle size is measured with a conventional method such as a number average particle size (an average of random 50 particles) derived from image analysis of transmission electron microscope images.

In the present invention, the titanium dioxide for white pigment is contained in the outer phase, i.e., the oil phase, of the water-in-oil emulsified sunscreen cosmetic.

The blend ratio of the titanium dioxide for white pigment having an average particle size of 180 nm or more is 0-1.0 wt% of the total amount of the water-in-oil emulsified sunscreen cosmetic.

That is, the water-in-oil emulsified sunscreen cosmetic of the present invention either does not contain the titanium dioxide for white pigment having an average particle size of 180 nm or more, or contains it in the amount of 1.0 wt% or less relative to the total amount of the water-in-oil emulsified sunscreen cosmetic.

### "Not comprising octylmethoxy cinnamate, octocrylene, or avobenzone"

For conventional sunscreen cosmetics, ultraviolet absorbents octylmethoxy cinnamate, octocrylene, and avobenzone are blended in for the UVA and UVB ultraviolet protection, but the present invention characteristically does not contain octylmethoxy cinnamate, octocrylene, or avobenzone.

The present invention can manifest superior UVA and UVB ultraviolet protection effects without containing these ultraviolet absorbents and thus lower eye irritation.

In addition to the aforementioned essential ingredients, surfactants (emulsifiers), oil components, and water necessary to prepare a water-in-oil emulsified sunscreen cosmetic are added to the water-in-oil emulsified sunscreen cosmetic of the present invention. Details are described below.

### "Surfactant (emulsifier)"

The surfactant used in the water-in-oil emulsified sunscreen cosmetic of the present invention is a polyoxyethylene methylpolysiloxane copolymer.

Examples of the aforementioned polyoxyethylene methylpolysiloxane copolymer include PEG-11 methyl ether dimethicone ("KF-6011" ; from Shin-Etsu Chemical Co., Ltd.), PEG-9 dimethicone ("KF-6013" ; from Shin-Etsu Chemical Co., Ltd.), PEG-3 ("KF-6015" ; from Shin-Etsu Chemical Co., Ltd.), PEG-9 methyl ether dimethicone ("KF-6016" ; from Shin-Etsu Chemical Co., Ltd.), PEG-10 dimethicone ("KF-6017" ; from Shin-Etsu Chemical Co., Ltd.), PEG-11 methyl ether dimethicone ("KF-6018" ; from Shin-Etsu Chemical Co., Ltd.), PEG-9 dimethicone ("KF-6019" ; from Shin-Etsu Chemical Co., Ltd.), and PEG-12 dimethicone ("SH3771M" , "SH3772M" , "SH3773M" , "SH3775M" ; from Dow Corning Toray Company Ltd.).

The blend ratio of the surfactant is determined as appropriate; it is 0.5-4 wt% relative to the total amount of the water-in-oil emulsified sunscreen cosmetic.

### "Oil components"

The selection of the oil component blended into the water-in-oil emulsified sunscreen cosmetic of the present invention is not limited in particular. Examples include: liquid fats and oils such as avocado oil, tsubaki oil, turtle fatty acid, macadamia nut oil, corn oil, mink oil, olive oil, rapeseed oil, egg yolk oil, sesame oil, persic oil, wheat germ oil, sasanqua oil, castor oil, linseed oil, safflower oil, cotton seed oil, evening primrose oil, perilla oil, soybean oil, peanut oil, tea seed oil, Japanese nutmeg oil, rice bran oil, Chinese gimlet oil, Japanese gimlet oil, jojoba oil, germ oil, and triglycerin, glycerin trioctanoate, and glycerin triisopalmitate;
solid fats and oils such as cacao butter, coconut oil, horse fat, hydrogenated coconut oil, palm oil, beef tallow, mutton tallow, hydrogenated beef tallow, palm kernel oil, lard, Japanese core wax nucleus oil, hydrogenated oil, Japanese core wax, and hydrogenated castor oil;
waxes such as beeswax, candelilla wax, cotton wax, carnauba wax, lanolin, lanolin acetate, liquid lanolin, sugar cane wax, lanolin fatty acid isopropyl ester, hexyl laurate, reduced lanolin, jojoba wax, hard lanolin, polyoxyethylene (hereafter referred to as P0E) lanolin alcohol ether, P0E lanolin alcohol acetate, lanolin fatty acid polyethylene glycol, and P0E hydrogenated lanolin ethyl alcohol ether;
hydrocarbons such as liquid petrolatum, ozocerite, squalene, paraffin, ceresin, squalane, petrolatum, and microcrystalline wax;
ester oils such as isopropyl myristate, cetyl octanoate, octyl dodecyl myristate, isopropyl palmitate, butyl stearate, hexyl laurate, myristyl myristate, decyl oleate, hexyl decyl dimethyloctanoate, cetyl lactate, myristyl lactate, lanolin acetate, isocetyl stearate, isocetyl isostearate, cholesteryl 12-hydroxystearate, ethylene glycol di(2-ethylhexanoate), dipentaerythritol fatty acid ester, n-alkylene glycol monoisostearate, neopentyl glycol dicaprate, diisostearyl malate, glyceryl di-2-heptylundecanoate, trimethylolpropane tri-2-ethylhexylate, trimethylolpropane triisostearate, pentaerythritol tetra-2-ethylhexylate, glycerin tri-2-ethylhexylate, trimethylolpropane triisostearate, cetyl 2-ethyl hexanoate, 2-ethylhexyl palmitate, glycerin trimyristate, tri-2-heptyl undecanoic acid glyceride, castor oil fatty acid methyl ester, oleyl oleate, acetoglyceride, 2-heptylundecyl palmitate, diisopropyl adipate, 2-octyldodecyl N-lauroyl-L-glutamate, di-2-heptylundecyl adipate, di-2-ethylhexyl sebacate, 2-hexyldecyl myristate, 2-hexyldecyl palmitate, 2-hexyldecyl adipate, diisopropyl sebacate, and 2-ethylhexyl succinate; higher fatty acids such as lauric acid, myristic acid, palmitic acid, stearic acid, behenic acid, oleic acid, 12-hydroxystearic acid, undecylenic acid, lanolin fatty acid, isostearic acid, linolic acid, linoleic acid, and eicosapentanoeic acid; straight chain or branched chain alcohols such as lauryl alcohol, cetyl alcohol, stearyl alcohol, behenyl alcohol, myristyl alcohol, oleyl alcohol, cetostearyl alcohol, mono stearyl glycerin ether (batyl alcohol), 2-decyltetradecynol, lanolin alcohol, cholesterol, phytosterol, hexyl dodecanol, isostearyl alcohol, and octyl dodecanol;
silicone oils such as dimethylpolysiloxane and methyl phenyl polysiloxane; and
perfluoropolyethers or perfluorocarbons such as perfluorohexane and tri-perfluoro-n-butyl amine.

Examples of oil components particularly preferable for the present invention include silicone oils.

The blend ratio of the oil component is determined as appropriate based on the product type of the water-in-oil emulsified sunscreen cosmetic, such as an emulsion or cream; it is preferably 15-45 wt% relative to the total amount of the water-in-oil emulsified sunscreen cosmetic.

### " Water"

For water, preferably used are ion exchanged water and purified water.

The blend ratio of water is determined as appropriate; it is 5-30 wt% relative to the total amount of the water-in-oil emulsified sunscreen cosmetic.

In addition to the aforementioned essential ingredients, other ingredients normally used in cosmetics can be blended as necessary in the water-in-oil emulsified sunscreen cosmetic of the present invention; examples of such ingredients include humectants, thickeners, alcohols, chelating agents, antioxidants, perfumes, various medicinal ingredients, preservatives, neutralizing agents, and pH adjustment agents; and the water-in-oil emulsified sunscreen cosmetic can be prepared with a conventional method.

Particularly, blending in 3-10 wt% of 1,3-butylene glycol relative to the total amount of the water-in-oil emulsified sunscreen cosmetic can maintain the stability and antiseptic properties without increasing eye irritation, which is very preferable.

### EXAMPLES

The present invention is described in detail below by referring to specific examples. The present invention is not limited to these examples. The blend ratios in Examples are in wt% units relative to the total amount of the water-in-oil emulsified sunscreen cosmetic.

Water-in-oil emulsified sunscreen cosmetics of the formulations shown in Table 1 were prepared and the following evaluation tests were conducted with a panel of 30 female specialists.

The water-in-oil emulsified sunscreen cosmetics of Examples and Comparative examples shown in Table 1 were applied on the skin of the female specialist panel (N=30) ; the makeup effect was evaluated in terms of three points, i.e., "ability to cover pigmented spots and freckles" , "uniform finish" , and "natural finish" , based on the following criteria.
"Ability to cover pigmented spots and freckles"
+2: 25 or more recognized the covering ability.
+1: 18-24 recognized the covering ability.
0: 12-17 recognized the covering ability.
-1: 6-11 recognized the covering ability.
-2: 5 or less recognized the covering ability.

### "Uniform finish"

+2: 25 or more recognized the uniformity.
+1: 18-24 recognized the uniformity.
0: 12-17 recognized the uniformity.
-1: 6-11 recognized the uniformity.
-2: 5 or less recognized the uniformity.

### "Natural finish"

+2: 25 or more recognized the naturalness.
+1: 18-24 recognized the naturalness.
0: 12-17 recognized the naturalness.
+1: 6-11 recognized the naturalness.
-2: 5 or less recognized the naturalness.

### "Eye irritation"

The water-in-oil emulsified sunscreen cosmetics of Examples and Comparative examples shown in Table 1 were intentionally applied on the outer corner of the eye and, 15 minutes later, evaluation was conducted based on the following criteria.
Yes: One or more of the 30 female panelists said there was eye irritation.
No: All of the 30 female panelists said there was no eye irritation.

**[Table 1]**

| | Example 1 | Example 2 | Comparative example 1 | Comparative example 2 | Comparative example 3 | Comparative example 4 |
|---|---|---|---|---|---|---|
| Silicone-coated rutile type crystallized titanium dioxide (needle-like, particle minor axis 15 nm major axis 50 nm) | 7 | - | 7 | | 2 | 13 |
| Silicone-coated rutile type crystallized titanium dioxide (near spherical clumps, average particle size 300 nm) | | | | | | 1 |
| (a) Aluminum stearate-coated rutile type crystallized titanium dioxide (near spherical clumps, average particle size 35 nm) | 7 | 9 | | | | |
| (c) Silicone-coated zinc oxide (Average particle size 30 nm) | 10 | 12 | 10 | 7 | 18 | 10 |
| Octylmethoxy cinnamate | | | | 7.5 | 7.5 | |
| Octocrylene | | | | 2 | 1 | |
| Hexyl diethylaminohydroxybenzoylbenzoate | | | | 2 | | |
| Dimethicodiethyl benzal malonate | | 3 | | | | |
| Methyl polymethacrylate powder | 5 | 5 | 5 | 5 | 5 | 5 |
| (b) Siliicone-coated red iron oxide | 0.65 | 0.65 | 0.65 | 0.65 | 0.65 | 0.65 |
| (b) Silicone-coated yellow iron oxide | 2.5 | 2.5 | 2.5 | 2.5 | 2.5 | 2.5 |
| (b) Silicone-coated black iron oxide | 0.03 | 0.03 | 0.03 | 0.03 | 0.03 | 0.03 |
| Ion-exchanged water | to100 | to 100 | to100 | to100 | to100 | to100 |
| Glycerin | 5 | 5 | 5 | 5 | 5 | 5 |
| 1,3-butylene glycol | 6 | 6 | 6 | 6 | 6 | 6 |
| Xylitol | 1 | 1 | 1 | 1 | 1 | 1 |
| Disteardimoniun hectorite | 0.5 | 0.5 | 0.5 | 0.5 | 0.5 | 0.5 |
| Polyether-modified silicone *1 | 3.5 | 3.5 | 3.5 | 3.5 | 3.5 | 3.5 |
| Triethylhexanoin | 5 | 5 | 5 | 5 | 5 | 5 |
| Low viscosity dimethicone (viscosity 5 mPa·s) | 3 | 3 | 3 | 3 | 3 | 3 |
| Methylphenylpolysiloxane | 1 | 1 | 1 | 1 | 1 | 1 |
| Cyclomethicone | 19 | 19 | 19 | 19 | 19 | 19 |
| Trimethylsiloxysilicic acid | 1 | 1 | 1 | 1 | 1 | 1 |
| Sodium L-glutamate | 0.5 | 0.5 | 0.5 | 0.5 | 0.5 | 0.5 |
| Disodium edetate | 0.1 | 0.1 | 0.1 | 0.1 | 0.1 | 0.1 |
| Makeup effect (Ability to cover pigmented spots and freckles) | +2 | +2 | 0 | -2 | -1 | +1 |
| Makeup effect (Uniform finish) | +1 | +2 | +1 | 0 | -1 | 0 |
| Makeup effect (Natural finish) | +1 | +2 | +2 | +1 | 0 | -1 |
| Eye irritation | No | No | No | Yes | Yes | No |

| | | | | | | |
|---|---|---|---|---|---|---|
| *1: Silicone KF6017 (from Shin-Etsu Chemical Co. Ltd.) | | | | | | |

The results of the aforementioned Table 1 indicate that Examples of the present invention manifest superior effects, i.e., superior makeup effects (ability to cover pigmented spots and freckles, uniform finish, and natural finish) and no eye irritation.

Next, the ultraviolet absorption effect (absorbance) in the UVA and UVB regions, 280-400 nm, was measured for the aforementioned Examples and Comparative examples.

For the measurement, 50 *µ* g of each sample of the aforementioned Examples and Comparative examples was applied uniformly on a PMMA film (5 cm x 5 cm) at a proportion of 2.0 mg/cm². After allowing it to stand for 15 minutes, the measurement was conducted with a spectrophotometer (Hitachi U-4100).

The results are shown in FIG. 1. As shown in FIG. 1, Example 1 and Example 2 of the present invention are superior in terms of the ultraviolet absorption effect in the UVA and UVB regions, compared with Comparative examples 1-3.

### INDUSTRIAL APPLICABILITY

The present invention can provide a water-in-oil emulsified sunscreen cosmetic that is applied on the face including the areas surrounding the eyes, wherein irritation to the eyes is minor, the UVB and UVA protection effect is high, and a superior makeup effect is also manifested.

## Claims

1. A water-in-oil emulsified sunscreen cosmetic comprising the following ingredients (a), (b), (c), and (d) in the outer phase, as well as (e) and (f), but not comprising octylmethoxy cinnamate, octocrylene, or avobenzone,
(a) 5-15 wt% of hydrophobized rutile type crystallized titanium dioxide having an average particle size of 30-80 nm,
(b) 0.1-10 wt% of iron oxide,
(c) 5-15 wt% of hydrophobized zinc oxide having an average particle size of 20-80 nm,
(d) 0-1.0 wt% of titanium dioxide for white pigment having an average particle size of 180nm or more,
(e) 0.5-4 wt% of a surfactant,
(f) 5-30 wt% of water,
wherein (e) the surfactant is a polyoxyethylene methylpolysiloxane copolymer, crystals of ingredient (a) are not cone-shaped but near spherical clumps, and the total blend ratio of ingredients (a) and (c) is 16-25 wt%.

2. The water-in-oil emulsified sunscreen cosmetic of claim 1 additionally comprising 3-10 wt% of (g) 1,3-butylene glycol.

3. The water-in-oil emulsified sunscreen cosmetic of claim 1 or 2 that exhibits low eye irritation, high UVB and UVA protection effects, and superior makeup effects.

4. The water-in-oil emulsified sunscreen cosmetic of any of claims 1-3 wherein said makeup effects are the ability to cover pigmented spots and freckles, and give a uniform finish, and a natural finish.

## Patentansprüche

1. Wasser-in-Öl-emulgiertes Sonnenschutzkosmetikum, umfassend die folgenden Bestandteile (a), (b), (c) und (d) in der äußeren Phase sowie (e) und (f), jedoch nicht umfassend Octylmethoxyzinnamat, Octocrylen oder Avobenzon,
(a) 5-15 Gew.-% hydrophobiertes, kristallisiertes Titandioxid vom Rutiltyp mit einer durchschnittlichen Teilchengröße von 30-80 nm,
(b) 0,1-10 Gew.-% Eisenoxid,
(c) 5-15 Gew.-% hydrophobiertes Zinkoxid mit einer durchschnittlichen Teilchengröße von 20-80 nm,
(d) 0-1,0 Gew.-% Titandioxid als Weißpgiment mit einer durschnittlichen Teilchengröße von 180 nm oder mehr,
(e) 0,5-4 Gew.-% eines Tensids,
(f) 5-30 Gew.-% Wasser,
worin (e) das Tensid ein Polyoxyethylen-Methylpolysiloxan-Copolymer ist, Kristalle von Bestandteil (a) nicht kegelförmig, sondern nahezu sphärische Klumpen sind, und der gesamte Mischungsanteil der Bestandteile (a) und (c) 16-25 Gew.-% beträgt.

2. Wasser-in-Öl-emulgiertes Sonnenschutzkosmetikum nach Anspruch 1, zusätzlich umfassend 3-10 Gew.-% an (g) 1,3-Butylenglykol.

3. Wasser-in-Öl-emulgiertes Sonnenschutzkosmetikum nach Anspruch 1 oder 2, welches eine geringe Augenirritation, hohe UVB- und UVA-Schutzeffekte und ausgezeichnete Makeup-Effekte aufweist.

4. Wasser-in-Öl-emulgiertes Sonnenschutzkosmetikum nach irgendeinem der Ansprüche 1-3, wobei die Makeup-Effekte die Fähigkeit sind, pigmentierte Flecken und Sommersprossen zu überdecken und ein einheitliches Finish sowie ein natürliches Finish zu ergeben.

## Revendications

1. Un produit cosmétique anti-solaire émulsionné eau-dans-huile comprenant les ingrédients (a), (b), (c) et (d) suivants dans la phase externe, aussi bien que (e) et (f), mais ne comprenant pas d'octylméthoxycinnamate, d'octocrylène ou d'avobenzone,
(a) 5 à 15% en poids de dioxyde de titane cristallisé de type rutile hydrophobisé ayant une taille moyenne de particule de 30 à 80 nm,
(b) 0,1 à 10% en poids d'oxyde de fer,
(c) 5 à 15% en poids d'oxyde de zinc hydrophobisé ayant un granulométrie moyenne de 20 à 80 nm
(d) 0 à 1,0% en poids de dioxyde de titane pour un pigment blanc ayant une taille moyenne de particule de 180 nm ou plus,
(e) 0,5 à 5% en poids d'un surfactant,
(f) 5 à 30% d'eau,
dans lequel (e) le surfactant est un copolymère de polyoxéthylene et de méthylpolysiloxane, les cristaux du composant (a) ne sont pas de forme conique mais des amas proche de spheres, et le ratio du mélange total des ingrédients (a) et (c) est de 16 à 25% en poids.

2. Le produit cosmétique anti-solaire émulsionné eau-dans-huile selon la revendication 1, en outre comprenant 3 à 10 % en poids de (g) 1,3-butylèneglycol.

3. Le produit cosmétique anti-solaire émulsionné eau-dans-huile selon la revendication 1 ou 2 qui présente une faible irritation des yeux, des effets de protection contre les UVA et les UVB puissants, et des effets de maquillage supérieurs.

4. Le produit cosmétique anti-solaire émulsionné eau-dans-huile selon l'une quelconque des revendications 1 à 3 dans lequel lesdits effets de maquillage sont l'habilité de couvrir des taches pigmentées et des taches de rousseur, et de présenter un finition uniforme, et une peau naturelle.
